(19) Europäisches Patentamt / European Patent Office / Office européen des brevets

(11) **EP 1 449 549 B1**

(12) **FASCICULE DE BREVET EUROPEEN**

(45) Date de publication et mention
de la délivrance du brevet:
**19.08.2015   Bulletin 2015/34**

(51) Int Cl.:
***A61M 1/36*** *(2006.01)*      *A61M 1/16* *(2006.01)*

(21) Numéro de dépôt: **04012686.4**

(22) Date de dépôt: **17.10.1997**

(54) **Procédé de détermination de la recirculation du sang dans un accès vasculaire**

Verfahren zur Bestimmung der Blutrezirkulation in einem vaskulären Zugang

Process for determining the recirculation of blood in a vascular access

(84) Etats contractants désignés:
**AT BE CH DE ES FR GB IT LI NL SE**

(30) Priorité:  **18.10.1996  IT TO960855**

(43) Date de publication de la demande:
**25.08.2004   Bulletin 2004/35**

(62) Numéro(s) de document de la (des) demande(s)
initiale(s) en application de l'article 76 CBE:
**97943094.9 / 0 886 529**

(73) Titulaire: **GAMBRO HOSPAL (Schweiz) AG
4051 Basel (CH)**

(72) Inventeurs:
• **Canini, Enrico
  41037 Montizzuolo di Mirandola (IT)**
• **Fava, Massimo
  41037 Mirandola (IT)**
• **Cavicchioli, Giovanni
  41036 Medolla (IT)**

(74) Mandataire: **Ponzellini, Gianmarco et al
PGA S.r.l.
Via Mascheroni, 31
20145 Milano (IT)**

(56) Documents cités:
**EP-A1- 0 399 083     EP-A1- 0 693 297
US-A- 5 312 550**

**Description**

La présente invention concerne un procédé de détermination de la recirculation du sang dans un accès vasculaire.

La présente invention s'applique en particulier à la détermination de la recirculation du sang dans un accès vasculaire lors d'un traitement de dialyse, que l'on prendra comme exemple non limitatif dans la suite de la description.

On sait que le sang se compose d'une partie liquide, le plasma sanguin, et d'une partie corpusculaire formée par des cellules dont les globules rouges qui contiennent l'hémoglobine. En cas d'insuffisance rénale, on trouve, en outre, dans le sang des substances indésirables de bas poids moléculaire qui peuvent être éliminées par un traitement dialytique réalisé au moyen d'un appareil de dialyse.

Les traitements de dialyse présentent une efficacité définie comme le rapport entre le volume de sang purifié au cours de la séance de dialyse et le volume sanguin total du patient.

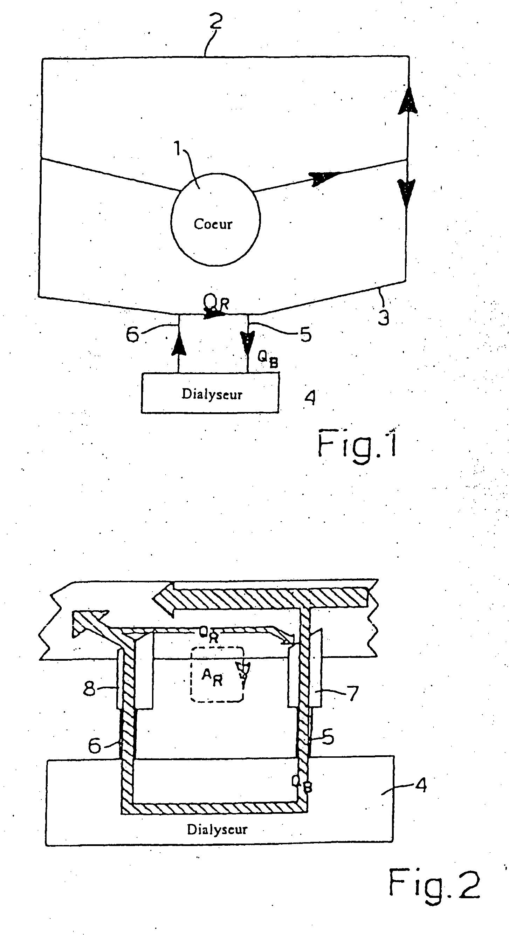

Un modèle simplifié des flux sanguins dans un système patient-circuit extracorporel où la communication entre le patient et le circuit extracorporel se fait au niveau d'un accès vasculaire de type fistule de Cimino-Brescia est illustré sur la figure 1, sur laquelle la référence 1 désigne le coeur, la référence 2 le circuit pulmonaire, la référence 3 le circuit vasculaire ou systémique et la référence 4 un dialyseur relié au circuit systémique 3 par l'intermédiaire d'une ligne de prélèvement de sang 5 (ligne artérielle) et d'une ligne de restitution de sang 6 (ligne veineuse).

Comme le montre la figure 1, le sang traité au cours d'une séance de dialyse provient du circuit systémique 3 dans lequel le sang s'écoule avec un débit limité. De ce fait, les traitements dialytiques actuels présentent une efficacité limitée et il n'existe pas à l'heure actuelle de moyens correcteurs permettant d'en augmenter la valeur.

Par ailleurs, l'efficacité des traitements dialytiques est également réduite par le phénomène connu dans le domaine médical sous le terme de "recirculation dans l'accès vasculaire", dont l'ampleur dépend de multiples facteurs tels que le débit du sang dans le circuit extracorporel, la position des aiguilles et le degré de sténose de la fistule. Il y a recirculation quand une partie du sang traité dans le circuit extracorporel est remise en circulation dans ce circuit par la ligne artérielle 5 aussitôt après avoir été injectée dans l'accès vasculaire 4 par la ligne veineuse 6. Ce phénomène est illustré sur la figure 2 où les références 7 et 8 désignent les aiguilles de prélèvement et de restitution du sang respectivement connectées aux lignes artérielle 5 et veineuse 6.

La valeur AR de la recirculation dans l'accès vasculaire est définie par l'expression suivante:

$$A_R\% = \frac{Q_R}{Q_B} \, 100 \qquad (1)$$

dans laquelle $Q_B$ est le débit du sang circulant dans le circuit extracorporel et $Q_R$ est le débit du sang retournant dans le circuit extracorporel par l'intermédiaire de la ligne artérielle 5 immédiatement après le traitement dialytique.

La connaissance de la valeur AR de la recirculation dans l'accès vasculaire présente plusieurs avantages pratiques en ce qui concerne les traitements de dialyse : elle indique que les aiguilles 7, 8 doivent être repositionnées lorsque la valeur AR de la recirculation est trop élevée et elle permet d'agir en vue d'augmenter la précision de la thérapie dialytique, de contrôler à long terme la sténose de la fistule et d'augmenter la durée de vie moyenne de la fistule elle-même.

Pour la détermination de la valeur AR de la recirculation dans l'accès vasculaire, on connaît plusieurs procédés de mesure qui peuvent être regroupés en deux grands groupes, le premier comprenant les procédés de mesure ne faisant pas appel à une sollicitation extérieure et le second faisant appel à une telle sollicitation.

Au premier groupe appartiennent les procédés de mesure qui ne font pas appel à une sollicitation de nature chimique ou physique du sang soumis au traitement de dialyse et qui ne font que quantifier des grandeurs physiologiques au cours de la séance de dialyse.

Appartient par exemple à ce groupe le procédé consistant à mesurer la concentration en urée de trois échantillons de sang prélevés au même moment dans la ligne artérielle, dans la ligne veineuse et dans le circuit vasculaire du patient, et à calculer la valeur AR de la recirculation dans l'accès vasculaire à partir de l'équation suivante (équivalente à l'équation 1) :

$$A_R\% = \frac{C_S - C_A}{C_S - C_V} \, 100 \qquad (2)$$

dans laquelle $C_S$ est la valeur de la concentration en urée dans la circulation vasculaire (concentration systémique), $C_A$ est la valeur de la concentration en urée dans la ligne artérielle (concentration artérielle) et $C_V$ est la valeur de la concentration en urée dans la ligne veineuse (concentration veineuse).

Ce procédé présente l'inconvénient de reposer sur l'hypothèse que, en l'absence de recirculation dans l'accès vasculaire, la valeur de la concentration systémique CS est égale à la valeur de la concentration artérielle CA. Or il a été démontré récemment qu'une telle hypothèse n'est pas valable dans toutes les conditions et qu'elle dépend du point de prélèvement. Il existe donc entre ces valeurs des différences qui compromettent la fiabilité de la mesure, même en l'absence de recirculation dans l'accès vasculaire.

Au second groupe appartiennent les procédés de mesure qui prévoient des sollicitations de nature chimique ou physique du sang soumis à un traitement de dialyse.

Appartient par exemple à ce deuxième groupe le procédé de mesure consistant, comme le précédent, à mesurer la concentration de l'urée dans le sang dans la ligne artérielle, dans la ligne veineuse et dans le circuit vasculaire du patient. Mais, à la différence du procédé mentionné ci-dessus, lors du prélèvement de sang dans la ligne artérielle pour la détermination de la valeur CS de la concentration systémique, le débit QB du sang circulant dans le circuit extracorporel est réglé au minimum afin de limiter au maximum la recirculation dans la fistule et donc de réduire les différences entre les valeurs CS et CA de la concentration systémique et de la concentration artérielle.

Appartient également au deuxième groupe le procédé consistant à imposer au sang du patient un traceur afin d'obtenir une dilution de nature chimique ou physique du sang et à contrôler simultanément, au moyen de capteurs spécifiques, l'évolution du sang dans la ligne artérielle, ou dans la ligne veineuse, ou dans les deux lignes. La comparaison des intégrales des signaux détectés par les capteurs permet de déterminer la valeur AR de la recirculation dans l'accès vasculaire.

En particulier, un premier procédé connu consiste à mesurer la température du sang au moyen de capteurs de température disposés sur la ligne veineuse et sur la ligne artérielle pour surveiller l'allure des températures relatives en réponse à une quantité de chaleur (considérée ici comme un traceur) administrée ou extraite du sang au moyen de l'appareil de dialyse.

Un second procédé de mesure connu fondé sur une dilution du sang est décrit dans le brevet US 5 312 550. Selon ce brevet, il est prévu d'injecter dans la ligne veineuse une substance ayant des propriétés physiques différentes de celles du sang et de détecter la recirculation du sang dans l'accès vasculaire au moyen d'une mesure des propriétés physiques de cette substance en amont du point d'injection de la substance.

Un troisième procédé de mesure connu fondé sur une dilution du sang est décrit dans le brevet US 5 510 717. Selon ce brevet, il est prévu d'injecter un bolus d'une solution hypertonique (traceur) dans la ligne veineuse, et de mesurer la conductivité du sang au moyen de deux capteurs disposés sur la ligne veineuse et sur la ligne artérielle afin de contrôler l'évolution de la conductivité relative en réponse à l'injection du bolus.

Un quatrième procédé de mesure connu fondé sur une dilution du sang consiste à injecter un bolus d'une solution isotonique (traceur) dans la ligne artérielle en amont d'un dispositif de mesure d'absorption optique disposé sur cette ligne. La valeur AR de la recirculation dans l'accès vasculaire est obtenue en comparant la mesure effectuée par le dispositif optique immédiatement après l'injection du bolus avec la mesure qui est effectuée après que le bolus a été partiellement remis en circulation dans le circuit extracorporel par l'accès artériel.

La présente invention a pour but de réaliser un procédé de détermination de la valeur de la recirculation du sang dans un circuit extracorporel qui soit simple, entièrement automatique et qui permette de réduire les erreurs de mesure et d'utiliser un petit nombre de capteurs, qui font déjà généralement partie d'un appareil de dialyse.

Pour atteindre ce but, on prévoit, conformément à l'invention un procédé de détermination de la recirculation du sang dans un accès vasculaire d'un patient dont le sang est soumis à traitement dans un appareil à membrane semi-perméable pour le traitement du sang relié au patient par un circuit extracorporel comprenant une ligne artérielle interconnectant l'accès vasculaire à une entrée de l'appareil de traitement, et une ligne veineuse interconnectant une sortie de l'appareil de traitement à l'accès vasculaire selon la revendication 1.

L'invention a aussi pour objet un système de traitement du sang selon la revendication 16.

D'autres caractéristiques et avantages de l'invention apparaîtront mieux à la lecture de la description qui va suivre. On se reportera aux dessins annexés, sur lesquels:

- la figure 1 représente un modèle simplifié des flux sanguins qui ont leur origine dans l'organisme d'un patient relié à un circuit pour circulation extracorporelle de sang;
- la figure 2 est un schéma simplifié d'un accès vasculaire et des flux sanguins qui y circulent;
- la figure 3 est un schéma simplifié d'un système de dialyse;
- la figure 4 est un organigramme des opérations effectuées par le système de la figure 3 pour mettre en oeuvre le procédé selon l'invention; et
- les figures 5, 6, 7 sont des diagrammes représentant l'évolution par rapport au temps de grandeurs mesurées ou contrôlées durant la mise en oeuvre du procédé selon l'invention.

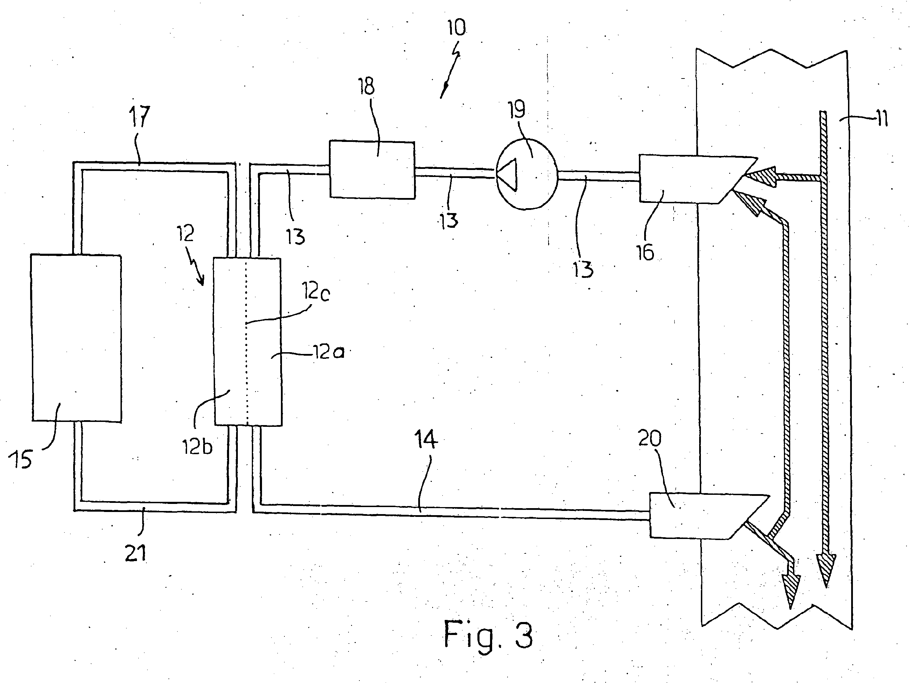

Sur la figure 3, la référence 10 désigne un circuit extracorporel pour le traitement du sang relié à une fistule 11 (accès vasculaire) d'un patient soumis à un traitement de dialyse. La figure 3 ne représente que les éléments d'un système de dialyse utiles à la compréhension du procédé selon l'invention.

Le circuit extracorporel 10 comprend un dialyseur 12 ayant un compartiment sang 12a et un compartiment pour liquide de dialyse 12b séparés par une membrane 12c semi-perméable. Une ligne artérielle 13 relie la fistule 11 du patient à une entrée du compartiment sang 12a et une ligne veineuse 14 relie une sortie du compartiment sang 12a à la fistule 11. La ligne artérielle 13 et la ligne veineuse 14 sont connectées à la fistule 11 par l'intermédiaire de canules 16, 20, la canule artérielle 16 étant implantée en amont de la canule veineuse 20 par rapport au sens de circulation du sang dans la fistule 11. Sur la ligne artérielle 13 sont disposées, dans le sens de circulation du sang, une pompe à sang 19 et un dispositif de mesure de l'hémoglobine 18.

Le compartiment pour liquide de dialyse 12b du dialyseur 12 a une entrée et une sortie reliées à une machine de dialyse 15, respectivement par l'intermédiaire d'une canalisation d'alimentation en liquide de dialyse frais 17 et une canalisation d'évacuation en liquide usé 21. La machine de dialyse 15 comprend notamment une pompe d'ultrafiltration (non représentée) permettant de provoquer le passage d'eau plasmatique au travers de la membrane 12c du dialyseur 12.

Le système de dialyse représenté sur la figure 3 comprend également une unité de calcul et de commande. Cette unité est reliée à une interface utilisateur par laquelle elle reçoit des instructions, telles que diverses valeurs de consigne. Par ailleurs, elle reçoit des informations émises par les organes de mesure du système, tel que le dispositif de mesure d'hémoglobine 18. Elle pilote, en fonction des instructions reçues et de modes d'opération et d'algorithmes programmés les organes moteurs du système, tels que la pompe à sang 19 et la pompe d'ultrafiltration.

La figure 3 met par ailleurs en évidence les flux de sang dans l'accès vasculaire.

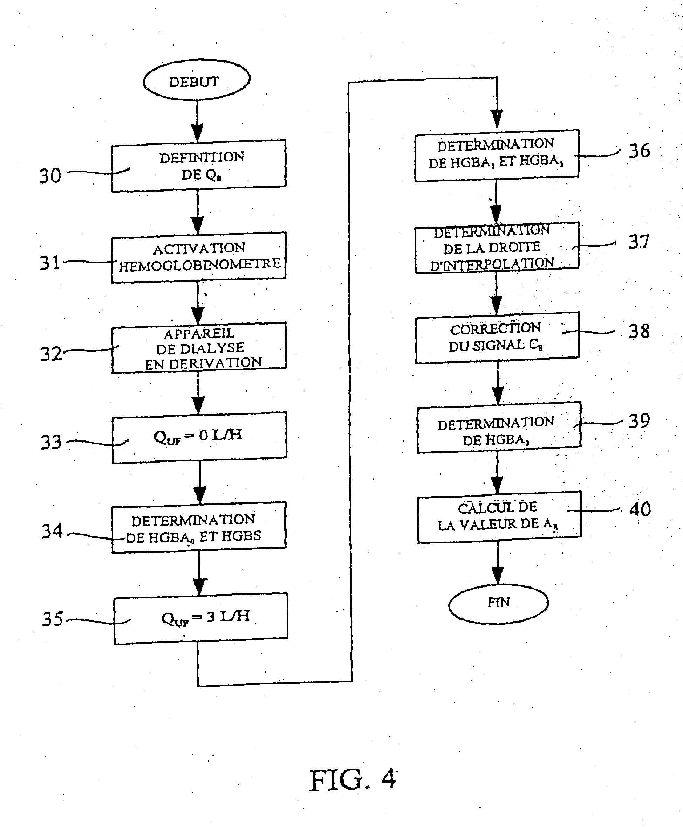

Lorsque le système de dialyse est en fonctionnement, les opérations décrites ci-dessous en référence à la figure 4 sont exécutées.

Le procédé de détermination de la recirculation du sang selon l'invention est fondé sur une variation d'une caractéristique du sang provoquée par la pompe d'ultrafiltration, à savoir la concentration du sang.

Au début de la séance de dialyse, comme le montre la figure 4, on impose une valeur QB au débit de la pompe à sang 19 (bloc 30).

L'hémoglobinomètre 18 est ensuite activé pour l'acquisition et la mémorisation du signal de concentration CE durant la totalité de la mesure nécessaire à la détermination de la recirculation (bloc 31).

Ensuite, la circulation du liquide de dialyse dans le dialyseur 12 est interrompue (bloc 32) de sorte qu'après une période transitoire initiale le liquide de dialyse dans le dialyseur a la même concentration électrolytique que le sang. La circulation du liquide de dialyse dans le dialyseur 12 n'est rétablie que lorsque les mesures nécessaire à la détermination de la recirculation ont été effectuées.

Cette opération permet de limiter l'influence indésirable des éventuels déséquilibres osmotiques sur la concentration en hémoglobine en raison desquels, même en l'absence d'ultrafiltration, il se produit en général une variation de la valeur de concentration en hémoglobine du sang due aux déplacements de l'eau contenue dans les cellules sanguines et dans les espaces interstitiels.

La pompe d'ultrafiltration est ensuite commandée pour que la valeur QUF du débit d'ultrafiltration dans le dialyseur 12 soit sensiblement nulle (bloc 33); il en résulte que les valeurs HGBA et HGBV des concentrations en hémoglobine dans la ligne artérielle 13 et dans la ligne veineuse 14 sont égales.

On détermine ensuite, en fonction du signal de concentration CE, la valeur instantanée HGBA0 de la concentration en hémoglobine dans la ligne artérielle 13, qui est égale à la valeur HGBS de la concentration systémique en hémoglobine lorsque le débit d'ultrafiltration est nul (bloc 34). On impose ainsi l'égalité suivante HGBS = HGBA0.

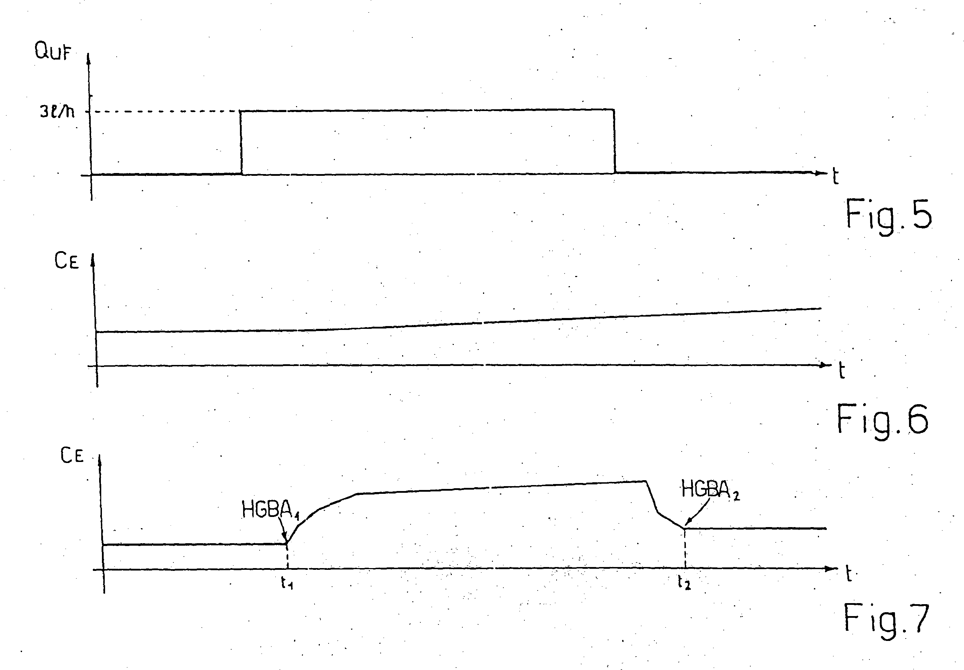

La pompe d'ultrafiltration est ensuite commandée pour que le débit d'ultrafiltration QUF soit égal à une valeur déterminée pendant un intervalle de temps déterminé. Par exemple, on impose un débit d'ultrafiltration QUF égal à 3 llh pendant 5 minutes (bloc 35); pour cet exemple, l'évolution du débit d'ultrafiltration QUF par rapport au temps est représentée sur le diagramme de la figure 5.

La première variation du débit d'ultrafiltration QUF, de zéro à la valeur déterminée, a pour conséquence une augmentation de la valeur de la concentration en hémoglobine du sang réintroduit dans la fistule 11 du patient par l'intermédiaire de la ligne veineuse 14, tandis que la seconde variation du débit d'ultrafiltration QUF, de la valeur déterminée à zéro, provoque une décroissance de la concentration en hémoglobine jusqu'à un niveau proche de la valeur initiale de l'hémoglobine.

A partir du signal de concentration CE fournit par l'hémoglobinomètre 18, il est possible de déduire la valeur de recirculation dans la fistule 11.

S'il n'y a pas de recirculation dans l'accès vasculaire, le sang concentré dans le dialyseur 12 lors de l'épisode d'ultrafiltration et injecté dans l'organisme du patient par l'intermédiaire de la ligne veineuse 14 parcourt un long trajet à l'intérieur du corps du patient avant de circuler à nouveau dans le dialyseur 12.

Le sang concentré est soumis durant ce trajet à une telle dilution que le signal de concentration CE produit par l'hémoglobinomètre 18 ne révèle initialement aucune augmentation de la concentration en hémoglobine dans la ligne artérielle 13 (comme le montre le diagramme de la figure 6), et il n'augmentera légèrement que quelques minutes après le début du palier de débit d'ultrafiltration (non illustré sur la figure 6 car extérieur à l'intervalle de temps représenté).

Si au contraire il y a recirculation dans l'accès vasculaire 11, une partie du sang concentré injecté dans le corps du patient est immédiatement réintroduit dans la ligne artérielle 13 et le signal de concentration CE fourni par l'hémoglobinomètre 18 augmente rapidement, comme le montre le diagramme de la figure 7. Dans l'exemple considéré, cette augmentation peut être observée environ une minute après la création d'un régime d'ultrafiltration et elle atteint une valeur sensiblement constante après environ trois minutes.

On calcule la valeur AR de la recirculation dans la fistule 11 à partir de la valeur du signal de concentration CE prise en régime stabilisé (blocs 36 à 40).

Le calcul de la valeur AR de la recirculation dans la fistule 11 est effectué en tenant compte du phénomène de "remplissage plasmatique" selon lequel, lorsque de l'eau plasmatique est soustraite du sang par ultrafiltration, l'organisme du patient soumis à ce traitement réagit et alimente le sang avec de l'eau prélevée dans les cellules du sang lui-même et dans les espaces interstitiels.

En effet, le signal de concentration CE produit par l'hémoglobinomètre 18 à la suite de la première variation du débit d'ultrafiltration comprend une composante principale correspondant à l'amplitude de l'hémofiltration imposée et une composante, croissant en général de manière linéaire dans le temps, correspondant à la différence entre le débit d'ultrafiltration et au débit du remplissage plasmatique.

Pour éliminer cette composante, en fonction de la valeur du signal de concentration CE mémorisé, on détermine les valeurs HGBA1 et HGBA2 prises par la concentration en hémoglobine dans la ligne artérielle 13 aux temps, notés t1 et t2 sur la figure 7, auxquels elle commence à augmenter au début de l'épisode d'ultrafiltration et finit de décroître à la fin de l'épisode d'ultrafiltration, respectivement (bloc 36).

Lorsque le débit de remplissage plasmatique est égal au débit d'ultrafiltration, les valeurs HGBA1 et HGBA2 sont égales. En revanche, les valeurs HGBA1 et HGBA2 sont différentes lorsque le débit de remplissage est nul ou est inférieur au débit d'ultrafiltration, HGBA2 étant supérieur à HGBA1.

On détermine alors l'équation de la droite d'interpolation passant par les points HGBA1 et HGBA2 de la figure 7, qui représente l'évolution par rapport au temps de la composante indésirable du signal de concentration CE, cette composante croissant généralement de façon linéaire en fonction du temps (bloc 37).

Ensuite, le signal de concentration CE est corrigé de l'effet du phénomène de remplissage plasmatique par soustraction de la droite d'interpolation au signal de concentration CE produit par l'hémoglobinomètre 18 pendant l'épisode d'hémofiltration et est mis en mémoire dans l'unité de commande et de calcul (bloc 38).

On détermine alors la valeur HGBA3 de la concentration en hémoglobine dans la ligne artérielle 13 en fonction de la valeur prise en régime établi par la concentration CE corrigée. Par exemple, cette valeur peut être la valeur mesurée à un instant déterminé (par exemple 3 minutes) suivant le début de l'épisode d'ultrafiltration (bloc 39).

La valeur HGBA3 de la concentration en hémoglobine dans la ligne artérielle 13 peut être aussi calculée de la façon suivante: à partir d'une série de valeurs de la concentration du sang échantillonnées à partir de t1, l'unité de calcul détermine l'équation de la courbe représentant l'évolution de la concentration du sang, puis en calcule l'asymptote. La concentration en hémoglobine HGBA3 est choisie égale à cette asymptote. L'intérêt de ce mode de détermination de HGBA3 est qu'il permet de réduire sensiblement la durée de l'épisode d'hémofiltration.

Enfin on calcule la valeur AR de la recirculation dans la fistule 11 à l'aide de l'équation suivante (bloc 40) :

$$A_R \% = \frac{Q_B - Q_{UF}}{Q_B + Q_{UF}\left(\dfrac{HGBS}{HGBA_3 - HGBS}\right)} \; 100$$

dans laquelle QB est le débit de la pompe à sang 19 (imposé au bloc 30), QUF est le débit d'ultrafiltration (imposé au bloc 35), HGBS est la concentration systémique (calculée au bloc 34) et HGBA3 est la concentration artérielle (calculée au bloc 39).

Les avantages du présent procédé de mesure sont les suivants.

Avant tout, le présent procédé est simple à mettre en oeuvre, contrairement aux procédés qui ne reposent pas sur une sollicitation extérieure, qui nécessitent des prélèvements sanguins et des examens de laboratoire, et aussi contrairement aux procédés qui reposent sur une sollicitation extérieure, qui nécessitent des interventions manuelles (injection de solution saline).

En outre, le présent procédé peut être mis en oeuvre de manière entièrement automatique.

En outre, le procédé utilise un seul capteur, l'hémoglobinomètre 18, au lieu de deux comme bon nombre des procédés décrits ci-dessus, et il permet donc de réduire les erreurs de mesure.

Enfin, le procédé ne nécessite aucune modification de l'appareil de dialyse 10 dans la mesure où les systèmes de dialyse courant comportent généralement un hémoglobinomètre 18 et une pompe d'ultrafiltration 15.

Finalement, il apparaît clairement qu'il est possible d'apporter du procédé décrit et illustré ici des modifications et des variantes sans pour autant sortir du cadre de la présente invention.

Par exemple, dans une variante du procédé, le débit d'ultrafiltration initial a une valeur déterminée différente de zéro et la variation du débit d'ultrafiltration provoquée pour mesurer la recirculation du sang dans l'accès vasculaire peut être positive ou négative (augmentation ou diminution du débit d'ultrafiltration d'une quantité déterminée).

Dans ce cas, le procédé prévoit:

- de mesurer la valeur instantanée HGBA0 de la concentration en hémoglobine dans la ligne artérielle 13 lorsque le débit d'ultrafiltration est égal à sa valeur déterminée initiale,
- de commander un palier négatif du débit d'ultrafiltration,
- de déterminer la valeur HGBA3 de la concentration en hémoglobine dans la ligne artérielle 13,
- d'égaler la valeur HGBS de la concentration systémique en hémoglobine à la valeur HGBA3 lorsque le débit d'ultrafiltration prend une valeur nulle au cours de cette étape (HGBS = HGBA3),
- de corriger cette valeur en fonction de la droite d'interpolation de la manière décrite ci-dessus, et
- de calculer la valeur AR de la recirculation dans l'accès vasculaire à l'aide de l'équation suivante:

## Revendications

1.  Procédé de détermination par une unité de calcul et de commande de la recirculation du sang dans un accès vasculaire (11) d'un patient dont le sang est soumis à un traitement par un système de traitement extracorporel de sang, ce dernier comprenant:

    - un dialyseur (12) à membrane semi-perméable pour le traitement du sang relié au patient par un circuit extracorporel ;
    - une ligne artérielle (13) interconnectant l'accès vasculaire (11) à une entrée du dialyseur (12),
    - une ligne veineuse (14) interconnectant une sortie du dialyseur (12) à l'accès vasculaire (11),
    - une pompe à sang (19) pour faire circuler le sang dans le dialyseur (12) ;
    - une pompe d'ultrafiltration pour provoquer une filtration d'eau plasmatique au travers de la membrane (12c) du dialyseur (12);
    - un capteur (18) pour déterminer un paramètre significatif de concentration du sang dans le dialyseur (12) disposés sur la ligne artérielle (13);
    - une unité de calcul et de commande pour recevoir des informations émises par le capteur (18).

    **caractérisé en ce que** l'unité de calcul et de commande permet notamment de:

    - commander la pompe à sang (19) pour faire circuler le sang du patient dans le dialyseur (12);
    - commander la pompe d'ultrafiltration pour provoquer, par filtration d'eau plasmatique au travers de la membrane, une variation momentanée de la concentration du sang dans le dialyseur (12) en réduisant ou en augmentant momentanément la proportion d'eau plasmatique du sang mis en circulation dans l'appareil à membrane;
    - mettre en mémoire les valeurs déterminées d'un paramètre significatif de la concentration du sang dans la ligne artérielle (13) mesuré par le capteur (18) disposés sur la ligne artérielle (13); et
    - calculer la recirculation du sang à partir des valeurs mesurées du paramètre.

2.  Procédé selon la revendication 1, **caractérisé en ce que** l'unité de calcul et de commande permet d'effectuer les étapes de:

    - commander la pompe à sang (19) pour régler le débit du sang dans le circuit extracorporel à une valeur déterminée $(Q_B)$;
    - déterminer une première valeur (HGBA0) du paramètre significatif de la concentration du sang dans le circuit extracorporel;
    - commander la pompe d'ultrafiltration pour provoquer une filtration d'eau plasmatique pour provoquer une première variation d'amplitude déterminée $(Q_{UF})$ du débit d'ultrafiltration dans le dialyseur (12);
    - déterminer une deuxième valeur (HGBA3) du paramètre significatif de la concentration du sang dans la ligne artérielle (13);
    - calculer la recirculation (AR) du sang dans le circuit extracorporel en fonction du débit du sang $(Q_B)$, de l'amplitude déterminée $(Q_{UF})$ de la première variation du débit d'ultrafiltration et des première et deuxième valeurs (HGBA0, HGBA3) du paramètre significatif de la concentration du sang dans la ligne artérielle (13).

**3.** Procédé selon la revendication 2, **caractérisé en ce que** l'étape de détermination d'une deuxième valeur (HGBA3) du paramètre significatif de la concentration du sang dans la ligne artérielle comprend les étapes de:

- commander la pompe d'ultrafiltration pour provoquer une deuxième variation du débit d'ultrafiltration dans l'appareil de traitement d'amplitude opposée à l'amplitude de la première variation;
- déterminer une troisième et une quatrième valeurs (HGBA1, HGBA2) du paramètre significatif de la concentration du sang dans la ligne artérielle (13) avant la première variation du débit d'ultrafiltration et après la deuxième variation du débit d'ultrafiltration, respectivement;
- déterminer une droite d'interpolation entre la troisième et la quatrième valeurs (HGBA1, HGBA2) du paramètre significatif de la concentration du sang;
- déterminer une valeur en régime établi du paramètre significatif de la concentration du sang dans la ligne artérielle après la première variation du débit d'ultrafiltration;
- corriger la valeur de régime établi du paramètre significatif de la concentration du sang dans la ligne artérielle (13) en fonction de la droite d'interpolation pour obtenir la deuxième valeur (HGBA3) du paramètre significatif de la concentration du sang dans la ligne artérielle.

**4.** Procédé selon la revendication 3, **caractérisé en ce que** l'unité de calcul et de commande permet de :

- déterminer la troisième valeur (HGBA31) du paramètre significatif de la concentration du sang dans la ligne artérielle (13) au moment où la concentration du sang commence à varier en réponse à la première variation du débit d'ultrafiltration et
- déterminer la quatrième valeur (HGBA2) du paramètre significatif de la concentration du sang dans la ligne artérielle (13) au moment où la concentration du sang finit de varier en réponse à la deuxième variation du débit d'ultrafiltration.

**5.** Procédé selon une des revendications 2 à 4, **caractérisé en ce que** l'unité de calcul et de commande permet de déterminer la deuxième valeur (HGBA3) du paramètre significatif de la concentration du sang dans la ligne artérielle (13) après un temps déterminé après le début de la première variation du débit d'ultrafiltration.

**6.** Procédé selon la revendication 5, **caractérisé en ce que** l'unité de calcul et de commande permet de déterminer la deuxième valeur (HGBA3) du paramètre significatif de la concentration du sang dans la ligne artérielle (13) en régime stabilisé après le début de la première variation du débit d'ultrafiltration.

**7.** Procédé selon une des revendications 2 à 6, **caractérisé en ce que** l'unité de calcul et de commande permet de régler le débit d'ultrafiltration dans le dialyseur sensiblement égal à zéro, préalablement à l'étape de provoquer une première variation du débit d'ultrafiltration..

**8.** Procédé selon la revendication 7, **caractérisé en ce que** l'unité de calcul et de commande permet de provoquer une première variation d'amplitude prédéterminée ($Q_{UF}$) du débit d'ultrafiltration dans le dialyseur par commande des moyens pour provoquer une filtration d'eau plasmatique au travers de la membrane (12c) pour augmenter le débit d'ultrafiltration.

**9.** Procédé selon la revendication 8, **caractérisé en ce que** l'unité de calcul et de commande permet de calculer la recirculation (AR) du sang dans le circuit extracorporel en résolvant l'équation suivante:

$$A_R\% = \frac{Q_B - Q_{UF}}{Q_B + Q_{UF}\left(\dfrac{HGBS}{HGBA_3 - HGBS}\right)} \cdot 100$$

où:

- $Q_B$ est le débit du sang dans le circuit extracorporel;
- $Q_{UF}$ est l'amplitude prédéterminée de la première variation du débit d'ultrafiltration ;
- HGBS est la valeur de la concentration systémique du sang, égale à la première valeur (HGBA0) du paramètre significatif de la concentration du sang dans la ligne artérielle (13); et
- HGBA3 est la deuxième valeur du paramètre significatif de la concentration du sang dans la ligne artérielle (13).

**10.** Procédé selon une des revendications 2 à 6, **caractérisé en ce que** l'unité de calcul et de commande permet de provoquer une première variation d'amplitude prédéterminée (QUF) du débit d'ultrafiltration dans le dialyseur par commande de la pompe d'ultrafiltration au travers de la membrane (12c) pour réduire un débit d'ultrafiltration existant jusqu'à ce qu'il devienne sensiblement égal à zéro.

**11.** Procédé selon la revendication 10, **caractérisé en ce que** l'unité de calcul et de commande permet de calculer la recirculation (AR) du sang dans le circuit extracorporel en appliquant la formule suivante:

$$A_R\% = \frac{Q_B - Q_{UF}}{Q_B + Q_{UF}(\frac{HGBS}{HGBA_0 - HGBS})} \, 100$$

où:

- $Q_B$ est le débit du sang dans le circuit extracorporel;
- $Q_{UF}$ est l'amplitude prédéterminée de la première variation du débit d'ultrafiltration;
- HGBA0 est la première valeur du paramètre significatif de la concentration du sang dans la ligne artérielle (13).
- HGBS est la valeur de la concentration systémique du sang, égale à la deuxième valeur (HGBA3) du paramètre significatif de la concentration du sang dans la ligne artérielle (13).

**12.** Procédé selon une des revendications 1 à 11, **caractérisé en ce que** :

- le dialyseur est un dialyseur (12) dans lequel un liquide de dialyse est mis en circulation, et **en ce que**,
- préalablement à l'étape de déterminer une première valeur du paramètre significatif de la concentration (HGBA0) du sang dans le circuit extracorporel, l'unité de calcul et de commande permet d'interrompre la circulation du liquide de dialyse.

**13.** Procédé selon une des revendications 1 à 12, **caractérisé en ce que** l'unité de calcul et de commande permet de ne rétablir la circulation du liquide de dialyse que lorsque les mesures nécessaires à la détermination de la recirculation ont été effectuées.

**14.** Procédé selon une quelconque des revendications 1 à 13 **caractérisé en ce que** l'unité de calcul et de commande contrôle la pompe d'ultrafiltration de sorte que le débit d'ultrafiltration $Q_{UF}$ soit égal à une valeur déterminée constante pendant un intervalle de temps déterminé.

**15.** Procédé selon une des revendications 1 à 14, **caractérisé en ce que** le paramètre significatif de la concentration du sang est la concentration du sang en hémoglobine.

**16.** Appareil de dialyse comprenant:

- un filtre dialyseur (12) à membrane semi-perméable pour le traitement du sang;
- une ligne artérielle destinée à interconnecter une fistule (11) d'un patient à une entrée du filtre dialyseur (12),
- une ligne veineuse (14) destinée à interconnecter une sortie du filtre dialyseur (12) à la fistule (11),
- une pompe d'ultrafiltration pour provoquer une filtration d'eau plasmatique au travers de la membrane du filtre dialyseur (12);
- un capteur (18) pour déterminer un paramètre indicatif de la concentration en hémoglobine du sang disposé sur la ligne artérielle (13):

  **caractérisé en ce que** le système de dialyse est programmé pour:

  - piloter la pompe pour réguler le flux d'ultrafiltration de façon à engendrer une variation de la concentration du sang dans le filtre dialyseur (12) en réduisant ou en augmentant la proportion d'eau plasmatique du sang mis en circulation dans l'appareil à membrane,
  - mettre en mémoire des valeurs du paramètre significatif de la concentration du sang, et
  - calculer la recirculation du sang à partir des valeurs du paramètre mesurées par le capteur (18) en réponse audit pilotage de la pompe (16).

**17.** Appareil selon la revendication précédente où le capteur (18) est un hémoglobinomètre.

**Patentansprüche**

**1.** Verfahren zur Bestimmung durch eine Rechen- und Steuereinheit der Blutrezirkulation in einem Gefäßzugang (11) eines Patienten, dessen Blut einer Behandlung durch ein extrakorporales Blutbehandlungssystem unterworfen ist, wobei das letztere umfasst:

- einen Dialysator (12) mit semipermeabler Membran zur Blutbehandlung, der durch einen extrakorporalen Kreislauf mit dem Patienten verbunden ist,
- eine arterielle Leitung (13), die den Gefäßzugang (11) mit einem Eingang des Dialysators (12) verbindet,
- eine venöse Leitung (14), die einen Ausgang des Dialysators (12) mit dem Gefäßzugang (11) verbindet,
- eine Blutpumpe (19) zum Zirkulieren des Blutes im Dialysator (12);
- eine Ultrafiltrationspumpe zum Bewirken einer Filtration von Plasmawasser durch die Membran (12c) des Dialysators (12);
- einen Sensor (18) zum Bestimmen eines signifikanten Parameters der Blutkonzentration im Dialysator (12), der an der arteriellen Leitung (13) angeordnet ist;
- eine Rechen- und Steuereinheit zum Empfangen von Informationen, die vom Sensor (18) ausgegebenen worden sind,

**dadurch gekennzeichnet, dass** die Rechen- und Steuereinheit ermöglicht:

- die Blutpumpe (19) zu steuern, dass das Blut des Patienten im Dialysator (12) zirkuliert;
- die Ultrafiltrationspumpe zu steuern, um durch die Filtration von Plasmawasser durch die Membran eine momentane Veränderung der Blutkonzentration im Dialysator (12) durch eine momentane Abnahme oder Zunahme des Anteils an Plasmawasser im in der Membranvorrichtung zirkulierenden Blut zu bewirken;
- die bestimmten Werte eines signifikanten Parameters der Blutkonzentration in der arteriellen Leitung (13), die vom an der arteriellen Leitung (13) angeordneten Sensor (18) gemessen wurden, zu speichern; und
- die Blutrezirkulation ausgehend von den gemessenen Werten des Parameters zu berechnen.

**2.** Verfahren nach Anspruch 1, **dadurch gekennzeichnet, dass** die Rechen- und Steuereinheit die Ausführung der Schritte ermöglicht:

- Steuern der Blutpumpe (19) zum Einstellen des Blutdurchsatzes im extrakorporalen Kreislauf auf einen bestimmten Wert ($Q_B$),
- Bestimmen eines ersten Wertes (HGBA0) des signifikanten Parameters der Blutkonzentration im extrakorporalen Kreislauf;
- Steuern der Ultrafiltrationspumpe zum Bewirken einer Filtration von Plasmawasser zum Bewirken einer ersten Veränderung der bestimmten Amplitude ($Q_{UF}$) des Ultrafiltrationsdurchsatzes im Dialysator (12);
- Bestimmen eines zweiten Wertes (HGBA3) des signifikanten Parameters der Blutkonzentration in der arteriellen Leitung (13);
- Berechnen der Rezirkulation (AR) des Bluts im extrakorporalen Kreislauf in Abhängigkeit des Blutdurchsatzes ($Q_B$), der bestimmten Amplitude ($Q_{UF}$) der ersten Veränderung des Ultrafiltrationsdurchsatzes und des ersten und zweiten Wertes (HGBA0, HGBA3) des signifikanten Parameters der Blutkonzentration in der arteriellen Leitung (13).

**3.** Verfahren nach Anspruch 2, **dadurch gekennzeichnet, dass** der Schritt des Bestimmens eines zweiten Wertes (HGBA3) des signifikanten Parameters der Blutkonzentration in der arteriellen Leitung die Schritte umfasst:

- Steuern der Ultrafiltrationspumpe zum Bewirken einer zweiten Veränderung des Ultrafiltrationsdurchsatzes in der Behandlungsvorrichtung mit einer zur Amplitude der ersten Veränderung entgegengesetzten Amplitude;
- Bestimmen eines dritten und eines vierten Wertes (HGBA1, HGBA2) des signifikanten Parameters der Blutkonzentration in der arteriellen Leitung (13) vor der ersten Veränderung des Ultrafiltrationsdurchsatzes bzw. nach der zweiten Veränderung des Ultrafiltrationsdurchsatzes;
- Bestimmen einer Interpolationsgeraden zwischen dem dritten und vierten Wert (HGBA1, HGBA2) des signifikanten Parameters der Blutkonzentration;
- Bestimmen eines Wertes im bestehenden Zustand des signifikanten Parameters der Blutkonzentration in der arteriellen Leitung nach der ersten Veränderung des Ultrafiltrationsdurchsatzes;
- Korrigieren des Wertes des bestehenden Zustands des signifikanten Parameters der Blutkonzentration in der arteriellen Leitung (13) in Abhängigkeit von der Interpolationsgeraden, um den zweiten Wert (HGBA3) des signifikanten Parameters der Blutkonzentration in der arteriellen Leitung zu erhalten.

4.  Verfahren nach Anspruch 3, **dadurch gekennzeichnet, dass** die Rechen- und Steuereinheit ermöglicht:

    - den dritten Wert (HGBA1) des signifikanten Parameters der Blutkonzentration in der arteriellen Leitung (13) zu bestimmen, wenn die Blutkonzentration sich in Reaktion auf die erste Veränderung des Ultrafiltrationsdurchsatzes zu verändern beginnt und
    - den vierten Wert (HGBA2) des signifikanten Parameters der Blutkonzentration in der arteriellen Leitung (13) zu bestimmen, wenn die Blutkonzentration in Reaktion auf die zweite Veränderung des Ultrafiltrationsdurchsatzes aufhört, sich zu verändern.

5.  Verfahren nach einem der Ansprüche 2 bis 4, **dadurch gekennzeichnet, dass** die Rechen- und Steuereinheit ermöglicht, den zweiten Wert (HGBA3) des signifikanten Parameters der Blutkonzentration in der arteriellen Leitung (13) nach einer bestimmten Zeit nach Beginn der ersten Veränderung des Ultrafiltrationsdurchsatzes zu bestimmen.

6.  Verfahren nach Anspruch 5, **dadurch gekennzeichnet, dass** die Rechen- und Steuereinheit ermöglicht, den zweiten Wert (HGBA3) des signifikanten Parameters der Blutkonzentration in der arteriellen Leitung (13) im stabilisierten Zustand nach Beginn der ersten Veränderung des Ultrafiltrationsdurchsatzes zu bestimmen.

7.  Verfahren nach einem der Ansprüche 2 bis 6, **dadurch gekennzeichnet, dass** die Rechen- und Steuereinheit ermöglicht, vor dem Schritt des Bewirkens einer ersten Veränderung des Ultrafiltrationsdurchsatzes den Ultrafiltrationsdurchsatz im Dialysator auf im Wesentlichen gleich null einzustellen.

8.  Verfahren nach Anspruch 7, **dadurch gekennzeichnet, dass** die Rechen- und Steuereinheit ermöglicht, eine erste Veränderung der vorbestimmten Amplitude ($Q_{UF}$) des Ultrafiltrationsdurchsatzes im Dialysator durch Steuerung der Mittel zum Bewirken einer Filtration von Plasmawasser durch die Membran (12c) zu bewirken, um den Ultrafiltrationsdurchsatz zu erhöhen.

9.  Verfahren nach Anspruch 8, **dadurch gekennzeichnet, dass** die Rechen- und Steuereinheit ermöglicht, die Rezirkulation (AR) des Bluts im extrakorporalen Kreislauf zu berechnen, indem die folgende Gleichung gelöst wird:

$$A_R\% = \frac{Q_B - Q_{UF}}{Q_B + Q_{UF}\left(\frac{HGBS}{HGBA_3 - HGBS}\right)} \, 100$$

wobei:

    - $Q_B$ der Blutdurchsatz im extrakorporalen Kreislauf ist,
    - $Q_{UF}$ die vorbestimmte Amplitude der ersten Veränderung des Ultrafiltrationsdurchsatzes ist,
    - HGBS der Wert der systemischen Blutkonzentration und gleich dem ersten Wert (HGBA0) des signifikanten Parameters der Blutkonzentration in der arteriellen Leitung (13) ist; und
    - HGBA3 der zweite Wert des signifikanten Parameters der Blutkonzentration in der arteriellen Leitung (13) ist.

10. Verfahren nach einem der Ansprüche 2 bis 6, **dadurch gekennzeichnet, dass** die Rechen- und Steuereinheit ermöglicht, eine erste Veränderung der vorbestimmten Amplitude ($Q_{UF}$) des Ultrafiltrationsdurchsatzes im Dialysator durch Steuerung der Ultrafiltrationspumpe durch die Membran (12c) zu bewirken, um einen bestehenden Ultrafiltrationsdurchsatz zu verringern, bis dieser im Wesentlichen gleich null wird.

11. Verfahren nach Anspruch 10, **dadurch gekennzeichnet, dass** die Rechen- und Steuereinheit ermöglicht, die Rezirkulation (AR) des Bluts im extrakorporalen Kreislauf zu berechnen, indem die folgende Gleichung angewandt wird:

$$A_R\% = \frac{Q_B - Q_{UF}}{Q_B + Q_{UF}\left(\frac{HGBS}{HGBA_0 - HGBS}\right)} \cdot 100$$

wobei:

- $Q_B$ der Blutdurchsatz im extrakorporalen Kreislauf ist,
- $Q_{UF}$ die vorbestimmte Amplitude der ersten Veränderung des Ultrafiltrationsdurchsatzes ist,
- HGBA0 der erste Wert des signifikanten Parameters der Blutkonzentration in der arteriellen Leitung (13) ist,
- HGBS der Wert der systemischen Blutkonzentration und gleich dem zweiten Wert (HGBA3) des signifikanten Parameters der Blutkonzentration in der arteriellen Leitung (13) ist.

12. Verfahren nach einem der Ansprüche 1 bis 11, **dadurch gekennzeichnet, dass**:

- der Dialysator ein Dialysator (12) ist, in dem eine Dialyseflüssigkeit in Zirkulation gebracht wird, und dadurch, dass
- vor dem Schritt des Bestimmens eines ersten Werts des signifikanten Parameters der Konzentration (HGBA0) des Bluts im extrakorporalen Kreislauf die Rechen- und Steuereinheit ermöglicht, die Zirkulation der Dialyseflüssigkeit zu unterbrechen.

13. Verfahren nach einem der Ansprüche 1 bis 12, **dadurch gekennzeichnet, dass** die Rechen- und Steuereinheit ermöglicht, die Zirkulation der Dialyseflüssigkeit wiederherzustellen, nur wenn die notwendigen Messungen zur Bestimmung der Rezirkulation durchgeführt worden sind.

14. Verfahren nach einem der Ansprüche 1 bis 13, **dadurch gekennzeichnet, dass** die Rechen- und Steuereinheit die Ultrafiltrationspumpe so steuert, dass der Ultrafiltrationsdurchsatz $Q_{UF}$ gleich einem bestimmten konstanten Wert während eines bestimmten Zeitintervalls ist.

15. Verfahren System nach einem der Ansprüche 1 bis 14, **dadurch gekennzeichnet, dass** der signifikante Parameter der Blutkonzentration die Blutkonzentration an Hämoglobin ist.

16. Dialysevorrichtung umfassend:

- einen Dialysatorfilter (12) mit semipermeabler Membran zur Blutbehandlung;
- eine arterielle Leitung (13) zur Verbindung einer Fistel (11) mit dem Eingang des Dialysatorfilters (12),
- eine venöse Leitung (14) zur Verbindung eines Ausgangs des Dialysatorfilters (12) mit der Fistel (11),
- eine Ultrafiltrationspumpe zum Bewirken einer Filtration von Plasmawasser durch die Membran des Dialysatorfilters (12);
- einen Sensor (18) zum Bestimmen eines signifikanten Parameters der Blutkonzentration an Hämoglobin, der an der arteriellen Leitung (13) angeordnet ist;

**dadurch gekennzeichnet, dass** das Dialysesystem programmiert ist zum:

• Steuern der Pumpe zur Einstellung des Ultrafiltrationsflusses, um eine Veränderung der Blutkonzentration im Dialysatorfilter (12) durch eine Abnahme oder Zunahme des Anteils an Plasmawasser in dem in der Membranvorrichtung zirkulierenden Blut zu bewirken,
• Speichern der Werte des signifikanten Parameters der Blutkonzentration, und
• Berechnen der Blutrezirkulation ausgehend von den vom Sensor (18) gemessenen Werten des Parameters in Reaktion auf die Steuerung der Pumpe (16).

17. Vorrichtung nach dem vorherigen Anspruch, wobei der Sensor (18) ein Hämoglobin-Messgerät ist.

**Claims**

1. A method for determining, by means of a computation and control unit, the blood recirculation in a vascular access (11) of a patient whose blood is subjected to a treatment by means of an extracorporeal blood treatment system, the latter comprising:

- a dialyzer (12) with semi-permeable membrane for blood treatment, connected to the patient by means of an extracorporeal circuit;
- an arterial line (13) interconnecting the vascular access (11) with an inlet of dialyzer (12),
- a venous line (14) interconnecting an outlet of the dialyzer (12) with the vascular access (11),
- a blood pump (19) for circulating blood in the dialyzer (12);
- a ultrafiltration pump for inducing a filtration of plasma water through the membrane (12c) of the dialyzer (12);
- a sensor (18) for determining a significant parameter of blood concentration in the dialyzer (12), arranged on the arterial line (13);
- a computation and control unit for receiving information sent by the sensor (18), **characterized in that** the computation and control unit specifically allows to:
- drive the blood pump (19) so as to circulate the patient's blood in the dialyzer (12);
- drive the ultrafiltration pump so as to induce by means of a filtration of plasma water through the membrane a momentary variation of blood concentration in the dialyzer (12) by momentarily reducing or increasing the proportion of blood plasma water circulating in the membrane apparatus,
- store the determined values of a significant parameter of blood concentration in the arterial line (13) as measured by the sensor (18) arranged on the arterial line (13); and
- calculate blood recirculation on the basis of the measured values of the parameter.

2. The method according to claim 1, **characterized in that** the computation and control unit allows to implement the following steps:

- driving the blood pump (19) for setting the blood flow rate in the extracorporeal circuit to a determined value ($Q_B$);
- determining a first value (HGBA0) of the significant parameter of blood concentration in the extracorporeal circuit;
- driving the ultrafiltration pump so as to induce a filtration of plasma water for inducing a first variation, with a given amplitude ($Q_{UF}$), of the ultrafiltration flow rate in the dialyzer (12);
- determining a second value (HGBA3) of the significant parameter of blood concentration in the arterial line (13);
- calculating blood recirculation (AR) in the extracorporeal circuit as a function of the blood flow rate ($Q_B$), of the given amplitude ($Q_{UF}$) of the first variation of the ultrafiltration flow rate, and of the first and second values (HGBA0, HGBA3) of the significant parameter of blood concentration in the arterial line (13).

3. The method according to claim 2, **characterized in that** the step of determining a second value (HGBA3) of the significant parameter of blood concentration in the arterial line comprises the following steps:

- driving the ultrafiltration pump for inducing a second variation of the ultrafiltration flow rate in the treatment apparatus, with an opposite amplitude to the amplitude of the first variation;
- determining a third and a fourth value (HGBA1, HGBA2) of the significant parameter of blood concentration in the arterial line (13) before the first variation of the ultrafiltration flow rate and after the second variation of the ultrafiltration flow rate, respectively;
- determining an interpolation line between the third and fourth values (HGBA1, HGBA2) of the significant parameter of blood concentration;
- determining a steady-state value of the significant parameter of blood concentration in the arterial line after the first variation of the ultrafiltration flow rate;
- correcting the steady-state value of the significant parameter of blood concentration in the arterial line (13) as a function of the interpolation line, in order to obtain the second value (HGBA3) of the significant parameter of blood concentration in the arterial line.

4. The method according to claim 3, **characterized in that** the computation and control unit allows to:

- determine the third value (HGBA1) of the significant parameter of blood concentration in the arterial line (13) at the time when blood concentration begins to vary in response to the first variation of the ultrafiltration flow rate, and
- determine the fourth value (HGBA2) of the significant parameter of blood concentration in the arterial line (13) at the time when blood concentration finishes varying in response to the second variation of the ultrafiltration flow rate.

5. The method according to one of the claims 2 to 4, **characterized in that** the computation and control unit allows to determine the second value (HGBA3) of the significant parameter of blood concentration in the arterial line (13) following a given delay after the start of the first variation of the ultrafiltration flow rate.

6. The method according to claim 5, **characterized in that** the computation and control unit allows to determine the second value (HGBA3) of the significant parameter of blood concentration in the arterial line (13) in a steady state after the start of the first variation of the ultrafiltration flow rate.

7. The method according to one of the claims 2 to 6, **characterized in that** the computation and control unit allows to set the ultrafiltration flow rate in the dialyzer substantially equal to zero, prior to the step of inducing a first variation of the ultrafiltration flow rate.

8. The method according to claim 7, **characterized in that** the computation and control unit allows to induce a first variation, with a pre-established amplitude ($Q_{UF}$), of the ultrafiltration flow rate in the dialyzer by driving the means for inducing a filtration of plasma water through the membrane (12c) so as to increase the ultrafiltration flow rate.

9. The method according to claim 8, **characterized in that** the computation and control unit allows to calculate blood recirculation (AR) in the extracorporeal circuit by solving the following equation:

$$A_R\% = \frac{Q_B - Q_{UF}}{Q_B + Q_{UF}\left(\frac{HGBS}{HGBA_3 - HGBS}\right)} \cdot 100$$

wherein:

- $Q_B$ is the blood flow rate in the extracorporeal circuit;
- $Q_{UF}$ is the pre-established amplitude of the first variation of the ultrafiltration flow rate;
- HGBS is the value of the systemic blood concentration, equal to the first value (HGBA0) of the significant parameter of blood concentration in the arterial line (13); and
- HGBA3 is the second value of the significant parameter of blood concentration in the arterial line (13).

10. The method according to one of the claims 2 to 6, **characterized in that** the computation and control unit allows to induce a first variation, with a pre-established amplitude ($Q_{UF}$), of the ultrafiltration flow rate in the dialyzer by driving the ultrafiltration pump through the membrane (12c) so as to reduce an existing ultrafiltration flow rate until it becomes substantially equal to zero.

11. The method according to claim 10, **characterized in that** the computation and control unit allows to calculate blood recirculation (AR) in the extracorporeal circuit by applying the following formula:

$$A_R\% = \frac{Q_B - Q_{UF}}{Q_B + Q_{UF}\left(\frac{HGBS}{HGBA_0 - HGBS}\right)} \cdot 100$$

wherein:

- $Q_B$ is the blood flow rate in the extracorporeal circuit;
- $Q_{UF}$ is the pre-established amplitude of the first variation of the ultrafiltration flow rate;
- HGBA0 is the first value of the significant parameter of blood concentration in the arterial line (13),
- HGBS is the value of the systemic blood concentration, equal to the second value (HGBA3) of the significant parameter of blood concentration in the arterial line (13).

12. The method according to one of the claims 1 to 11, **characterized in that**:

- the dialyzer is a dialyzer (12) in which a dialysis liquid is be circulated, and **in that**
- prior to the step of determining a first value of the significant parameter of blood concentration (HGBA0) in the extracorporeal circuit, the computation and control unit allows to interrupt the circulation of the dialysis liquid.

**13.** The method according to one of the claims 1 to 12, **characterized in that** the computation and control unit allows to re-establish the circulation of the dialysis liquid only when the measurements needed for determining the recirculation have been made.

**14.** The method according to one of the claims 1 to 13, **characterized in that** the computation and control unit controls the ultrafiltration pump so that the ultrafiltration flow rate $Q_{UF}$ corresponds to a given constant value during a given time interval.

**15.** The method according to one of the claims 1 to 14, **characterized in that** the significant parameter of blood concentration is the blood hemoglobin concentration.

**16.** A dialysis apparatus comprising:

- a dialyzer filter (12) with semi-permeable membrane for blood treatment;
- an arterial line intended to interconnect a patient's fistula (11) with an inlet of dialyzer filter (12),
- a venous line (14) intended to interconnect an outlet of the dialyzer filter (12) with the fistula (11),
- a ultrafiltration pump for inducing a filtration of plasma water through the membrane of the dialyzer filter (12);
- a sensor (18) for determining a significant parameter of blood hemoglobin concentration, arranged on the arterial line (13);

**characterized in that** the dialysis system is programmed for:

• driving the pump for setting the ultrafiltration flow so as to generate a variation of blood concentration in the dialyzer filter (12) by reducing or increasing the proportion of blood plasma water circulating in the membrane apparatus,
• storing values of the significant parameter of blood concentration, and
• calculating blood recirculation on the basis of the values of the parameter as measured by the sensor (18) in response to said driving of the pump (16).

**17.** The apparatus according to the preceding claim, wherein the sensor (18) is a hemoglobin meter.

$$A_R\% = \frac{Q_B - Q_{UF}}{Q_B + Q_{UF}\left(\frac{HGBS}{HGBA_0 - HGBS}\right)} \cdot 100$$

En outre, le present procédé peut être aussi mis en oeuvre, avec une précision comparable, lorsque le phénomène de remplissage plasmatique donne lieu, du fait de divers facteurs tels que les mouvements et les déséquilibres hydriques et osmotiques, à un apport d'eau dans le sang superieur à l'eau plasmatique ultrafiltrée, cest-à-dire à une diminution de la concentration en hémoglobine dans le sang. Dans ce cas, la composante indésirable du signal de concentration produit par l'hémoglobinomètre 18 présente une allure décroissant de façon linéaire dans le temps.

Fig.1

Fig.2

Fig. 3

EP 1 449 549 B1

FIG. 4

QuF

3ℓ/h

t

EP 1 449 549 B1

Fig.5

CE

t

Fig.6

CE

HGBA₁

HGBA₂

t₁          t₂          t

Fig.7

**EP 1 449 549 B1**

## RÉFÉRENCES CITÉES DANS LA DESCRIPTION

### Documents brevets cités dans la description

- US 5312550 A
- US 5510717 A